**Europäisches Patentamt**

**European Patent Office**

**Office européen des brevets**

(19)

(11) Veröffentlichungsnummer: **0 194 466**

**A2**

# EUROPÄISCHE PATENTANMELDUNG

(12)

(21) Anmeldenummer: **86101946.1**

(22) Anmeldetag: **15.02.86**

(51) Int. Cl.⁴: **A 61 K 7/00**
**A 61 K 9/00**

(30) Priorität: **13.03.85 DE 3508928**

(43) Veröffentlichungstag der Anmeldung:
**17.09.86 Patentblatt 86/38**

(84) Benannte Vertragsstaaten:
**AT BE CH DE FR GB IT LI LU NL SE**

(71) Anmelder: **Giulini Chemie GmbH**
**Giulinistrasse 2**
**D-6700 Ludwigshafen/Rhein(DE)**

(72) Erfinder: **Völler, Heinrich, Dr.Dipl.-Chem.**
**Kelterstrasse 34**
**D-7401 Pliezhausen(DE)**

(72) Erfinder: **Wallner, Angelika**
**Leipzigerstrasse 10**
**D-6834 Ketsch(DE)**

(74) Vertreter: **Benatzky, Erika, Dr.**
**Giulinistrasse 2 Postfach 150480**
**D-6700 Ludwigshafen/Rh.(DE)**

(54) Kombination zur antimikrobiellen und antioxidativen Stabilisierung von Externa und Körperpflegemitteln.

(57) Kombination zur antimikrobiellen und antioxidativen Stabilisierung von Externa und Körperpflegemitteln, bestehend aus:

| | |
|---|---|
| 15 - 30 Gew.% | Methylparaben (PHB-Methylester) |
| 5 - 10 Gew.% | Propylparaben (PHB-Propylester) |
| 20 - 35 Gew.% | Propylenglykol |
| 25 - 35 Gew.% | Triethylenglykol |
| 0 - 5 Gew.% | α- Tocopherolacetat |
| 0 - 25 Gew.% | Imidazolidinyl-Harnstoff |
| 0 - 20 Gew.% | 2-Phenoxyethanol |
| 0 - 5 Gew.% | Buthylhydroxytoluol (BHT) |
| 0 - 10 Gew.% | Isothiazolinone |
| 0 - 1,5 Gew.% | $MgCl_2.6H_2O$ und/oder $Cu(NO_3)_2.3H_2O$ |
| 0,1 - 1,0 Gew.% | Buthylhydroxyanisol (BHA) |
| 0,1 - 1,0 Gew.% | Ascorbylpalmitat |

EP 0 194 466 A2

Kombination zur antimikrobiellen und
antioxidativen Stabilisierung von Externa und Körperpflegemitteln

Gegenstand der vorliegenden Erfindung ist eine neue, Methylparaben, Propylparaben und Propylenglykol enthaltende Kombination zur antimikrobiellen und antioxidativen Stabilisierung von Externa und Körperpflegemitteln, insbesondere auf wäßriger Basis, sowie ein Verfahren zu ihrer Herstellung.

Es ist seit langem bekannt, daß Bakterien, Hefen und Schimmelpilze organische Materialien während längeren Stehens, insbesondere bei Feuchtigkeits- und Luftzutritt, in unerwünschter Weise verändern. Diese Zersetzung von Eiweißen, Fetten und Kohlenhydraten kann man schon äußerlich durch das Auftreten von Trübungen, Färbungen, fremden Geruch, Gärung oder Schimmelpilzrasen erkennen. Sie beeinträchtigt aber nicht nur das äußere Bild, sondern vermindert auch die Qualität des betreffenden Stoffes. Dabei können die Mikroorganismen sogar gesundheitsschädigende Substanzen produzieren. Ein Konservierungsmittel hat die Aufgabe, diese durch Kleinlebewesen verursachten, unerwünschten Veränderungen zu verhindern oder zu verzögern.

Die Ester der p-Hydroxybenzoesäure, allgemein als Parabene (HBE) bezeichnet, sind wegen ihrer bakteriziden und fungostatischen Wirkung auf eine Zahl von Mikroorganismen ausgezeichnete Konservierungs- und Sterilhaltungsmittel. Sie sind vor allem im sauren und neutralen, aber

- 2 -

auch im alkalischen Milieu wirksam und nahezu ungiftig. Sie geben bereits in niedrigen Konzentrationen eine ausreichenden Schutz. Wegen ihrer
Farb- und Geschmacklosigkeit haben sie schnell
Eingang in die Lebensmittel, pharmazeutische und
kosmetische Industrie gefunden; sie werden für
viele technische Zwecke und in den letzten Jahren
verstärkt als Arzneimittel ( pilzbekämpfende Therapie ) benutzt. Zwei der HBE wurden sogar in die
bekanntesten Pharmacopöen aufgenommen (BP, USP
und DAB).

Chemisch verhalten sich die Ester der p-Hydroxybenzoesäure bei ihrer Verwendung weitgehend indifferent und zeigen selbst keine störenden Nebenerscheinungen wie Färbungen, Trübungen u.ä. Es sind
feine, weiße Pulver. Sie werden als Methyl-,
Äthyl-, n-Butyl- und Propyl-Ester (Methylparaben,
Äthylparaben, Butylparaben und Propylparaben )
eingesetzt.

Die Löslichkeit des Methylparabens in Wasser ist
jedoch gering, und reicht für manche Konservierungszwecke nicht aus. Obwohl die Parabene unter
Bildung eines Metallsalzes in Alkalilaugen sehr
gut löslich sind, finden sie in dieser Form nur
in Sonderfällen Verwendung. Im alkalischen Milieu
sind die Parabene nämlich nur mäßig beständig.
Sie verseifen, besonders in der Wärme, leicht.

Es ist weiterhin bekannt, daß organische Materialien, vor allen Dingen Fette und Öle, einem oxidativen Angriff durch Luftsauerstoff ausgesetzt
sind. Zur Verhinderung des oxidativen Abbaues

- 3 -

werden Oxidationsinhibitoren und/oder Antioxidantien eingesetzt. Beispielhaft wird an dieser Stelle eine Mischung, bestehend aus 10 Teilen Butylhydroxytoluol (BHT), 1 bis 5 Teilen Butylhydroxyanisol (BHA) und 1 bis 2 Teilen Ascorbylpalmitat, genannt.

Auch sind bereits Kombinationen von Imidazolidinyl-Harnstoff und Parabenen als wirksame Konservierungssysteme gegen Bakterien, Hefen und Schimmel vorgeschlagen worden (J.Soc. Cosmetic Chemists 28 ( 1977), 83/87. Imidazolidinyl-Harnstoff ist die Bezeichnung für eine bakterizide Verbindung, deren Herstellung z.B. in der US-PS 3 248 285 beschrieben ist. Als Vertreter von Imidazolidinyl-Harnstoffverbindungen sind z.Zt. u.a. auf dem Markt: Germall 115, Germall II, Euxyl K 200, Unicide U-13 und Biopure 100. Im Gegensatz zu den vorerwähnten Parabenen, die in der Ölphase leicht und in der Wasserphase schwerlöslich sind, können Imidazolidinyl-Harnstoffverbindungen in Wasser jedoch leicht gelöst werden. Wegen dieser Löslichkeitsunterschiede ist der Einsatz von Kombinationen aus Imidazolidinyl-Harnstoff und Parabenen in Externa und Hautpflegemitteln schwierig und problematisch dann, wenn ihr Einsatz in Produkten auf wäßriger Basis, beispielsweise in Emulsionen, vorgesehen ist.

Die zur Produktstabilisierung gegen mikrobiellen und oxidativen Verderb bestimmten Komponenten des Stabilisierungssystems werden daher in Körperpflegemittel vom Emulsionstyp, bestimmte Cremes und Lotions, in separaten Schritten eingearbeitet.

— 4 —

So werden beispielsweise Propylparabenen, Butylhydroxyanisol und Butylhydroxytoluol in der Ölphase der Emulsion gelöst, hingegen Methylparaben und Imidazolidinyl-Harnstoff in der wäßrigen Phase. Ein derartiges Stabilisierungsverfahren ist sowohl kostenintensiv als auch zeitaufwendig, da es unter Erhitzen und intensiver Mischung durchgeführt werden muß.

Es ist auch schon vorgeschlagen worden, die einzelnen Komponenten des Stabilisierungssystems jeweils in einem wasserlöslichen Alkohol, z.B. Propylenglykol und Triethylenglykol, zu lösen und die erhaltenen " Vorlösungen " in das Körperpflegemittel einzuarbeiten. Anschließend wird der Imidazolidinyl-Harnstoff in der wäßrigen Phase der Emulsion aufgelöst. Auch dieses Verfahren ist kosten- und zeitaufwendig.

Es hat sich weiterhin herausgestellt, daß die auf dem Markt befindlichen antimikrobiellen und antioxidativen Stabilisatoren bzw. Kombinationen dieser Verbindungen manche Externa und Körperpflegemittel nicht ausreichend stabilisieren. Sie können in verschiedenen Mitteln ihre Wirkung nicht voll entfalten, insbesondere zeigen viele nur ein begrenztes Wirkungsspektrum, sind also nicht imstande sowohl grampositive als auch gramnegative Bakterien, Hefen und Schimmel an der Vermehrung zu hemmen bzw. abzutöten.

Aufgabe der vorliegenden Erfindung war es daher,

- 5 -

eine Kombination von antimikrobiellen und antioxidativen Stabilisatoren zu finden, die sowohl
ein erweitertes Wirkungsspektrum zeigt, insbesondere gegen das gesamte Spektrum von Mikroorganismen wirksam ist, als auch einen oxidativen
Abbau verhindert. Auch sollen die eingangs erwähnten Schwierigkeiten behoben werden, die Kombinationen sollen sich besonders leicht in Emulsionen und Mittel auf Wasserbasis einarbeiten
lassen.

Die Lösung der gestellten Aufgabe basiert auf der
Erkenntnis, daß eine Kombination von Stabilisatoren in Externa und Körperpflegemitteln anders
wirksam sein kann als der einzelne Stabilisator
allein.

Erfindungsgemäß wird die gestellte Aufgabe mit
einer Kombination gelöst, die aus

| | | |
|---|---|---|
| 15 - 30 | Gew.% | Methylparaben (PHB-Methylester) |
| 5 - 10 | " | Propylparaben (PHB-Propylester) |
| 20 - 35 | " | Propylenglykol |
| 25 - 35 | " | Triethylenglykol |
| 0 - 5 | " | $\alpha$-Tocopherolacetat |
| 0 - 25 | " | Imidazolidinyl-Harnstoff |
| 0 - 20 | " | 2-Phenoxyethanol |
| 0 - 5 | " | Butylhydroxytoluol (BHT) |
| 0 - 10 | " | Isothiazolinone |
| 0 - 1,5 | " | $MgCl_2 \cdot 6 H_2O$ und/oder $Cu(NO_3)_2 \cdot 3 H_2O$ |
| 0,1- 1,0 | " | Butylhydroxyanisol ( BHA) |
| 0,1- 1,0 | " | Ascorbylpalmitat |

besteht.

- 6 -

Bevorzugte erfindungsgemäße Zusammensetzungen enthalten:

|  |  |  |
|---|---|---|
| 18 - 25 | Gew.% | Methylparaben |
| 5 - 8 | " | Propylparaben |
| 23 - 29 | " | Propylenglykol |
| 28 - 32 | " | Triethylenglykol |
| 10 - 15 | " | Imidazolidinyl-Harnstoff |
| 2 - 4 | " | Butylhydroxytoluol |
| 1 - 2 | " | Isothiazolinone (mit 13% Aktivgehalt ) |
| 0,3- 1,5 | " | $MgCl_2 \cdot 6\ H_2O$ und/oder $Cu(NO_3)_2 \cdot 3\ H_2O$ |
| 0,2- 0,9 | " | Butylhydroxyanisol |
| 0,1- 0,7 | " | Ascorbylpalmitat |

Für besonders hitzeintensive Herstellungsverfahren eignen sich Kombinationen, bestehend aus

|  |  |  |
|---|---|---|
| 18 - 25 | Gew.% | Methylparaben |
| 6 - 10 | " | Propylparaben |
| 21 - 29 | " | Propylenglykol |
| 27 - 35 | " | Triethylenglykol |
| 13 - 18 | " | 2-Phenoxyethanol |
| 0,2- 0,6 | " | Butylhydroxyanisol |
| 0,5- 1,5 | " | ɑ- Tocopherolacetat |
| 0,3- 1,0 | " | Ascorbylpalmitat |

sehr gut.

Daß Isothiazolinone in so geringer Konzentration in den Parabene und Glykole enthaltenden Kombinationen gemäß vorliegender Erfindung eine derart effektive Wirkung gegen das gesamte Spektrum von Mikroorganismen zeigen, muß als überraschend bezeichnet werden und war nicht vorhersehbar. Überraschend war auch, daß sowohl Isothiazolinone als auch 2-Phenoxyethanol mit Antioxidantien so erfolgreich kombiniert werden kann.

Die neuen Kombinationen lassen sich leicht in Externa und Körperpflegemittel auf Wasserbasis einarbeiten, wobei beim Einarbeiten von Isothiazolinone enthaltenden Kombinationen allerdings Temperaturen oberhalb 40°C vermieden werden sollten. Temperaturen von 35 - 40°C sind anwendbar. Bei Temperatureinwirkung von über 40°C über einen längeren Zeitraum verlieren nämlich diese Kombinationen deutlich an Wirkung. Es empfiehlt sich daher in solchen Fällen Isothiazolinone in Kombinationen durch 2-Phenoxyethanol zu ersetzen oder zusammen mit 2-Phenoxyethanol einzusetzen.

Die neuen Kombinationen werden zweckmäßigerweise so hergestellt, daß Triethylenglykol und Propylenglykol in einem Reaktionsgefäß auf Temperaturen von 35 bis 80°C erhitzt werden, gegebenenfalls in diesem Temperaturbereich mit Imidazolidinyl-Harnstoff vermischt und nach Abkühlung auf mindestens 45°C mit den restlichen Komponenten vermischt und auf Raumtemperatur abgekühlt wird. Die neuen Kombinationen sind in Mengen bis zu etwa 1,5 Gew.% in 60° heißem Wasser gut dispergierbar, wobei Mengen von 1 Gew.%, bezogen auf das Externum oder Körperpflegemittel, ausreichend sind. Es hat sich allerdings gezeigt, daß auch geringere Mengen bereits voll wirksam sein können. Die erforderlichen Mengen werden in Abhängigkeit von der Zusammensetzung der Externa und Körperpflegemittel bestimmt.

- 8 -

Der Vorteil der neuen, Methylparaben, Propylparaben und Propylenglykol enthaltenden Kombinationen zur antimikrobiellen und antioxidativen Stabilisierung von Körperpflegemitteln, wird vor allem darin gesehen, daß die hierfür erforderlichen Komponenten nunmehr in einem einzigen Arbeitsgang in die Körperpflegemittel und Externa eingearbeitet werden können, eine Vielzahl von Einzelschritten damit entfällt. Dadurch wird die Herstellung der Körperpflegemittel wesentlich erleichtert. Außerdem wird die notwendige homogene Verteilung der Komponenten in den Externa und Körperpflegemittel viel rascher erzielt. Die dadurch erzielte Zeitersparnis ist beträchtlich.

Wie sich überraschenderweise weiterhin gezeigt hat, zeigen die neuen Kombinationen eine synergistische Wirkung. Es wird vermutet, daß die effektive, sich synergistisch verstärkende Wirkung u.a. eine Folge der homogenen Verteilung ist. Da Wirkungsuntersuchungen und Konzentrationsuntersuchungen gezeigt haben, daß die Komponenten in den erfindungsgemäßen Konzentrationsbereichen sowohl optimale Löslichkeiten als auch Wirkungen zeigen, wird vermutet, daß die synergistische Wirkung auch eine Folge der außerordentlich guten Löslichkeit ist.

Schließlich ist an dieser Stelle noch erwähnenswert, daß eine fehlerfreie Dosierung mit den neuen Kombinationen wesentlich leichter ist als eine fehlerfreie Dosierung mit vielen Einzelkomponenten. Die Auffindbarkeitskontrolle der Einzelkomponenten im Endprodukt ist durch Leitsub-

- 9 -

stanzen, z.B. 2-Phenoxyethanol, möglich, was
ebenfalls vorteilhaft ist. Außerdem wird durch
die Kombinationen die Lagerhaltung erleichtert
bzw. vereinfacht.

Bei der Herstellung von Emulsionen können die
Kombinationen kurz nach der Vermischung der Fett-
und Wasserphase eingerührt werden. Lösungvermittler und andere Hilfsmittel sind in der Regel beim
Einemulgieren der neuen Kombinationen nicht erforderlich. Werden jedoch die neuen Kombinationen
in wasserfreien oder rein wäßrigen Präparationen
eingesetzt, ist die zusätzliche Verwendung eines üblichen Emulgators bzw. Lösungsvermittlers zweckmäßig.

Anhand der nachstehenden Beispiele und Diagramme
soll nun der Gegenstand der Erfindung näher erläutert werden.

Beispiel 1:
31 kg Triethylenglykol und 25,7 kg Propylenglykol
werden in einem heiz- und kühlbaren Rührkessel auf
70°C erhitzt. Zu dieser heißen Lösung gibt man
15 kg Imidazolidinyl-Harnstoff und 2 kg BHT bis
zur vollständigen Lösung langsam zu. Dann kühlt
man die erhaltene klare Lösung auf 45°C ab und
rührt nacheinander 20 kg Methylparaben, 5,2 kg Propylparaben, 0,2 kg BHA, 0,2 kg Ascorbylpalmitat
und 1,0 kg Isothiazolinone ein. Anschließend wird
zur Wirkungsstabilisierung noch 1,5 kg $Cu(NO_3)_2 \cdot 3 H_2O$ ( in anderen Formulierungen wurden anstelle
von $Cu(NO_3)_2 \cdot 3 H_2O$  600 g $MgCl_2 \cdot 6 H_2O$) zugegeben. Zum Schluß wird auf Zimmertemperatur abgekühlt.

– 10 –

Beispiel 2:

35 kg Triethylenglykol  und 27,7 kg Propylenglykol
werden wie im Beispiel 1 erhitzt. In die Lösung
werden

| 2,0 | kg | BHT |
| 25,0 | kg | Methylparaben |
| 10,0 | kg | Propylparaben |
| 0,2 | kg | BHA |
| 0,1 | kg | Ascorbylpalmitat |

nacheinander eingerührt und auf RT abgekühlt.

Beispiel 3:

Wie im Beispiel 2 werden die nachstehenden Komponenten
miteinander vermischt und zwar:

| 27 | kg | Triethylenglykol |
| 21,0 | kg | Propylenglykol |
| 15 | kg | 2-Phenoxyethanol |
| 25 | kg | Methylparaben |
| 10 | kg | Propylparaben |
| 0,5 | kg | BHA |
| 1 | kg | $\alpha$-Tocopherolacetat  und |
| 0,5 | kg | Ascorbylpalmitat |

Mikrobiologischer Konservierungsmittel-Belastungstest:

Die mit den Konservierungsmitteln versetzten Proben wurden bis zur Kontamination mit den Test-Bakterienstämmen und während des Belastungstests bei Zimmertemperatur gelagert.

Die Prüfung der antimikrobiellen Wirksamkeit wurde mit den Stämmen:

und  Staphylococcus aureus        ATCC  6538
     Pseudomonas aeruginosa       ATCC  3027

durchgeführt.

Methodik:

18 Stunden bei $37^{o}$C in Casein-Soja-Bouillon inkubierte Kulturen wurden mit steriler phosphatgepufferter physiologischer Kochsalzlösung so eingestellt, daß nach gleichmäßiger Vermengung mit destilliertem Wasser eine mikrobielle Belastungskonzentration von $10^6$ bis $10^7$ lebenden Keimen pro ml Probematerial resultierte. Sterile Probenahmen erfolgten aus Wasser nach 3, 24 und 48 Stunden bzw. nach 7 Tagen und bei Creme-Proben nach 1, 2, 7, 14, 21 und 28 Tagen; die Proben wurden unmittelbar untersucht.

Die Keimzahlen wurden mit dem Plattengußverfahren unter Verwendung von Konservierungsmittel-Antagonisten bestimmt. Dazu wurden log 10-Verdünnungsreihen in sterilisierter Casein-Pepton-Lezithin-Polysorbat-Bouillon hergestellt (Firma Merck). Zur Verbesserung der Löslichkeit wurde zusätzlich 1% sterilisiertes Tween 80 beigemengt.

- 12 -

Pro Verdünnungsstufe wurde jeweils 1 ml in 2 Casein-
Soja-Agarplatten gegossen und bei 37°C 72 bis 96 Stunden
bebrütet, damit auch verzögert wachsende Kolonien erfaßt
werden konnten.

Gleichzeitig wurden Anreicherungen in antagonistenhaltiger
Bouillon mit anschließender Subkultur zur Prüfung auf
Keimfreiheit durchgeführt.

Nach dieser Methode wurden die gemäß den Beispielen 1
und 2 hergestellten Kombinationen getestet. Das mit den
erfindungsgemäßen Kombinationen erzielte ausgezeichnete
Ergebnis kann den beiliegenden 4 Diagrammen entnommen
werden.

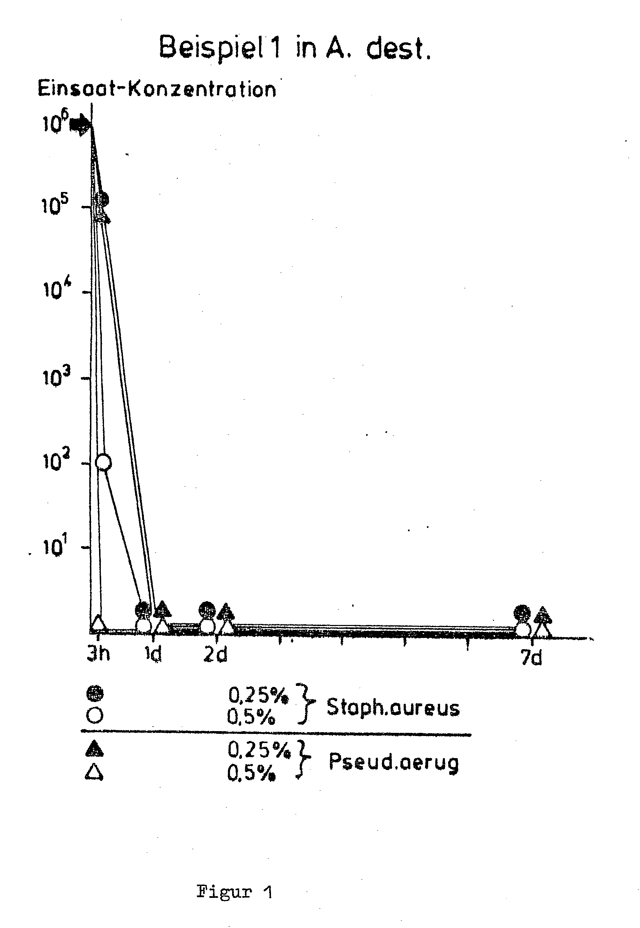

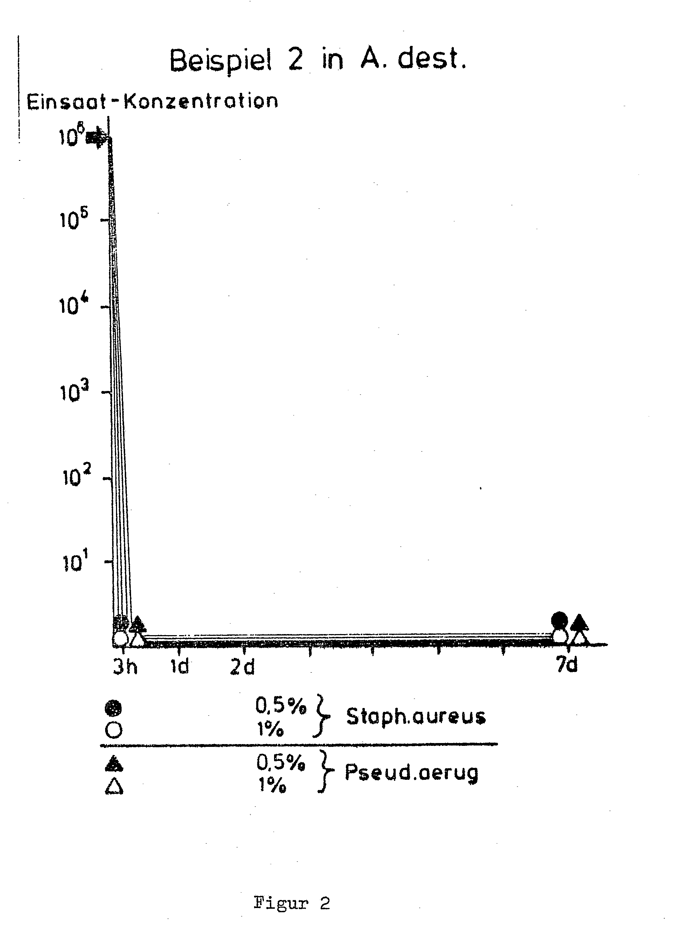

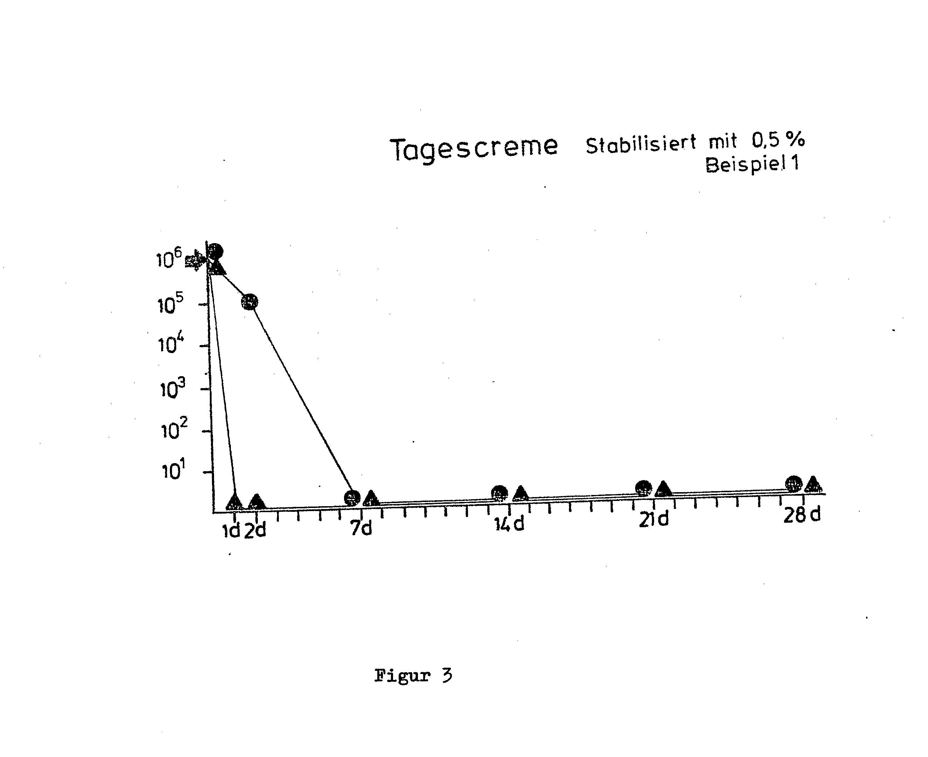

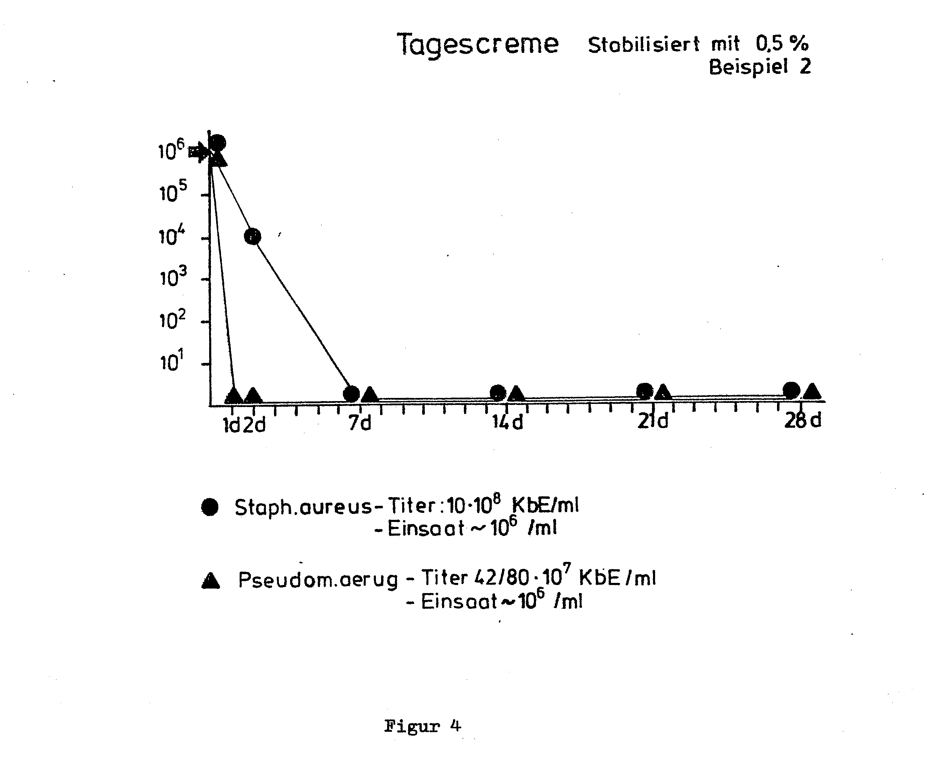

Patentansprüche

1) Methylparaben, Propylparaben und Propylenglykol enthaltende Kombination zur antimikrobiellen und antioxidativen Stabilisierung von Körperpflegemitteln, dadurch gekennzeichnet, daß sie folgende Komponenten enthält:

| | | |
|---|---|---|
| 15 - 30 Gew.% | Methylparaben (PHB-Methylester) |
| 5 - 10 " | Propylparaben (PHB-Propylester) |
| 20 - 35 " | Propylenglykol |
| 25 - 35 " | Triethylenglykol |
| 0 - 5 " | α -Tocopherolacetat |
| 0 - 25 " | Imidazolidinyl-Harnstoff |
| 0 - 20 " | 2-Phenoxyethanol |
| 0 - 5 " | Butylhydroxytoluol (BHT) |
| 0 - 10 " | Isothiazolinone |
| 0 - 1,5 " | $MgCl_2 \cdot 6H_2O$ und/oder $Cu(NO_3)_2 \cdot 3H_2O$ |
| 0,1- 1,0 " | Buthylhydroxyanisol (BHA) |
| 0,1- 1,0 " | Ascorbylpalmitat |

2) Kombination zur antimikrobiellen und antioxidativen Stabilisierung von Körperpflegemitteln nach Anspruch 1, dadurch gekennzeichnet, daß sie folgende Komponenten enthält:

| | | |
|---|---|---|
| 18 - 25 Gew.% | Methylparaben |
| 5 - 8 " | Propylparaben |
| 23 - 29 " | Propylenglykol |
| 28 - 32 " | Triethylenglykol |
| 10 - 15 " | Imidazolidinyl-Harnstoff |
| 2 - 4 " | Butylhydroxytoluol |
| 1 - 2 " | Isothiazolinone(mit 13% Aktivgehalt) |

0,3 - 1,5  Gew.%  $MgCl_2$ . 6 $H_2O$ und/oder

$Cu(NO_3)_2$ . 3 $H_2O$

0,2 - 0,9  Gew.% Butylhydroxyanisol

0,1 - 0,7  "      Ascorbylpalmitat

3) Kombination zur antimikrobiellen und anti-oxidativen Stabilisierung von Körperpflege-mitteln nach Anspruch 1, dadurch gekennzeichnet, daß sie folgende Komponenten enthält:

18 - 25  Gew.%  Methylparaben

6 - 10  "      Propylenparaben

21 - 29  "      Propylenglykol

27 - 35  "      Triethylenglykol

13 - 18  "      2-Phenoxyethanol

0,2- 0,6  "      Butylhydroxyanisol

0,5- 1,5  "      α- Tocopherolacetat

0,3- 1,0  "      Ascorbylpalmitat

4) Verfahren zur Herstellung von Kombinationen nach den Ansprüchen 1 bis 3, dadurch gekenn-zeichnet, daß Triethylenglykol und Propylen-glykol in einem Reaktionsgefäß auf Temperaturen von 35 bis 80°C erhitzt werden, gegebenenfalls in diesem Temperaturbereich mit Imidazolidinyl-Harnstoff vermischt und nach Abkühlung auf mindestens 45°C mit den restlichen Komponenten vermischt und auf Raum-temperatur abgekühlt werden.

# Beispiel 1 in A. dest.

Einsaat-Konzentration

| | | |
|---|---|---|
| ● | 0,25% | Staph.aureus |
| ○ | 0,5% | |
| ▲ | 0,25% | Pseud.aerug |
| △ | 0,5% | |

Figur 1

Figur 2

Tagescreme  Stabilisiert mit 0,5 %
Beispiel 1

Figur 3

Tagescreme  Stabilisiert mit 0,5 %
Beispiel 2

● Staph.aureus-Titer:$10 \cdot 10^8$ KbE/ml
  -Einsaat ~$10^6$ /ml

▲ Pseudom.aerug - Titer $42/80 \cdot 10^7$ KbE/ml
  -Einsaat ~$10^6$ /ml

Figur 4